# EUROPÄISCHE PATENTSCHRIFT

(11) **EP 2 146 580 B1**
(45) Veröffentlichungstag und Bekanntmachung des Hinweises auf die Patenterteilung: **19.10.2011**
(21) Anmeldenummer: 08773342.4
(22) Anmeldetag: 14.05.2008
(51) Int. Cl.: A01N 59/00, A01N 25/22, A01P 1/00, C02F 1/46, C02F 1/461, C02F 1/467, C25B 1/26

(54) **VERWENDUNG EINES DESINFEKTIONSMITTELS AUF DER BASIS WÄSSRIGER, HYPOCHLORIGE SÄURE (HOCl) ENTHALTENDER LÖSUNGEN**
USE OF A DISINFECTANT BASED ON AQUEOUS, HYPOCHLOROUS ACID (HOCl)-CONTAINING SOLUTIONS
L'UTILISATION D'UN AGENT DE DESINFECTION À BASE DE SOLUTIONS AQUEUSES CONTENANT DE L'ACIDE HYPOCHLORIQUE (HOCL),

(30) Priorität: 15.05.2007 DE 102007022994
(43) Veröffentlichungstag der Anmeldung: 27.01.2010
(73) Patentinhaber: Actides Gmbh, 69518 Abtsteinach (DE)
(72) Erfinder: BELTRUP, Alfons, 69 509 Mörlenbach (DE); FÜCHTJOHANN, Lars, 58769 Nachrodt-Wiblingwerde (DE); GROSS, Steven, 64743 Beerfelden (DE); JÖST, Bernd, 69518 Abtsteinach (DE)
(74) Vertreter: Lenz, Steffen
(86) Internationale Anmeldenummer: PCT/EP2008/003863
(87) Internationale Veröffentlichungsnummer: WO 2008/138601

(56) Entgegenhaltungen:
- WO-A-01/00030
- WO-A-01/28336
- US-A- 4 287 079
- US-A- 4 561 994
- DATABASE WPI Week 200539 Thomson Scientific, London, GB; AN 2005-380319 XP002510085 & JP 2005 150216 A (HITACHI CABLE LTD) 9. Juni 2005 (2005-06-09)
- DATABASE WPI Week 200607 Thomson Scientific, London, GB; AN 2006-062652 XP002510086 & JP 2006 012980 A (HITACHI CABLE LTD) 12. Januar 2006 (2006-01-12)
- NAKAGAWARA S ET AL: "Spectroscopic characterization and the pH dependence of bactericidal activity of the aqueous chlorine solution" ANALYTICAL SCIENCES, JAPAN SOCIETY FOR ANALYTICAL CHEMISTRY, TOKYO, JP, Bd. 14, Nr. 4, 1. August 1998 (1998-08-01), Seiten 691-698, XP002974725 ISSN: 0910-6340

## Beschreibung

Die Erfindung betrifft die Verwendung einer wäßrigen, hypochlorige Säure (HOCl) enthaltenden Lösung als Desinfektionsmittel.

Desinfektionsmittel auf der Basis von hypochloriger Säure sind bekannt, wobei hypochlorige Säure eine desinfektiös höchst wirksame Substanz darstellt und insbesondere auch gegenüber ihren Salzen, den Hypochloriten (OCl⁻), welche ihrerseits desinfektiös wirksam sind, eine erheblich bessere Desinfektionswirkung besitzt. Zur Herstellung von Desinfektionsmitteln auf der Basis von hypochloriger Säure/Hypochlorit ist neben anderen Darstellungsweisen das Verfahren der elektrochemischen Aktivierung bekannt, welches vornehmlich zur Desinfektion von Wasser eingesetzt wird. Hierbei wird eine verdünnte Lösung eines Elektrolyts, insbesondere eines Neutralsalzes, wie Natriumchlorid (NaCl) bzw. Kochsalz, Kaliumchlorid (KCl) oder dergleichen, in einem Elektrolysereaktor durch Anlegen einer Spannung an dessen Elektroden in einen zur Desinfektion geeigneten, aktiven Zustand überführt, welcher in aller Regel metastabiler Natur ist und je nach Art des Wassers und den eingestellten Verfahrensparametern über längere Zeit andauern kann. Ein solches Verfahren der elektrochemischen Aktivierung (ECA) ist beispielsweise in der zum Prioritätsdatum der vorliegenden Anmeldung noch nicht veröffentlichten WO 2007/093385 A1 beschrieben. Der bei diesem Verfahren eingesetzte Elektrolysereaktor weist einen Kathodenraum mit einer oder mehreren Kathoden sowie einen Anodenraum mit einer oder mehreren Anoden auf, wobei der Anodenraum und der Kathodenraum mittels eines elektrisch leitfähigen - insbesondere eines für Ionen leitfähigen - Diaphragmas bzw. mittels einer Membran mit den genannten Eigenschaften räumlich voneinander getrennt sind, um eine Vermischung der in beiden Räumen befindlichen Wasser-/Elektrolytlösung zu verhindern.

Während bei der Elektrolyse, wie der Chlor-Alkali-Elektrolyse, in der Regel ein im wesentlichen vollständiger Umsatz der eingesetzten Edukte - im Falle des Einsatzes einer Natriumchloridlösung zu Chlorgas (Cl₂) und Natronlauge (Na-OH), im Falle des Einsatzes einer Kaliumchloridlösung zu Chlorgas und Kalilauge (KOH) - unter Einsatz hoch konzentrierter Elektrolytlösungen angestrebt wird, um vornehmlich die Chlorgasausbeute zu maximieren, wird die Wasser-/Elektrolytlösung bei der elektrochemischen Aktivierung demgegenüber in erheblich verdünnterer Form, in der Regel in einer Konzentration von maximal etwa 20 g/l, vorzugsweise maximal etwa 10 g/l, insbesondere zwischen 0,1 g/l und 10 g/l oder zwischen 0,1 g/l und 5 g/l oder auch nur zwischen 0,1 g/l und 5 g/l, dem Elektrolysereaktor aufgegeben und lediglich zu einem sehr geringen Anteil umgesetzt, um die physikalischen und chemischen Eigenschaften der Lösung in vorteilhafter Weise zu verändern und insbesondere das Redoxpotential des mit dem Elektrolyt versetzten Wassers zu erhöhen, wodurch eine desinfizierende Wirkung erhalten wird. Entsprechend werden die Reaktionsbedingungen, wie Druck, Temperatur, Elektrodenstrom etc., bei der elektrochemischen Aktivierung im allgemeinen moderater gewählt als bei der Chlor-Alkali-Elektrolyse, welche in der Regel vor allem bei erhöhter Temperatur im Bereich zwischen 50°C und 90°C durchgeführt wird, während die elektrochemische Aktivierung bei Raumtemperatur durchgeführt werden kann. Von Vorteil bei der elektrochemischen Aktivierung ist insbesondere die gute Gesundheits- und Umweltverträglichkeit der anläßlich der elektrochemischen Aktivierung erzeugten Stoffe in deren jeweiligen Konzentrationen, welche gemäß der deutschen Trinkwasserverordnung (TrinkwV) auch zugelassen und als Desinfektionsmittel wirksam sind.

Wie bei der Elektrolyse findet auch bei der elektrochemischen Aktivierung an der Anode (d.h. an der positiv geladenen Elektrode) eine Oxidation statt, während an der Kathode (d.h. an der negativ geladenen Elektrode) eine Reduktion stattfindet. Beim Einsatz einer verdünnten Neutralsalzlösung, wie einer Natriumchloridlösung, wird an der Kathode gemäß der nachfolgenden Reaktionsgleichung (1) vornehmlich Wasserstoff erzeugt:

2 H₂O + 2 e⁻ ---> H₂ + 2 OH⁻ (1),

welcher nach Ausgasen aus der Lösung z.B. aus dem Kathodenraum des Reaktors abgeführt wird. Darüber hinaus wird die verdünnte Wasser-/Elektrolytlösung in dem Kathodenraum des Elektrolysereaktors durch die Bildung von Hydroxidionen alkalisch.

An der Anode werden gemäß den nachfolgenden Reaktionsgleichungen (2) und (3) insbesondere die chemischen Oxidationsmittel Sauerstoff (O₂) und Chlor (Cl₂) erzeugt, welche bekanntermaßen hinsichtlich einer Desinfektion von Wasser wirksam sind. Ferner ist zu beachten, daß infolge der Bildung von H₃O⁺-Ionen die verdünnte Wasser-/Elektrolytlösung in dem Anodenraum des Elektrolysereaktors sauer wird:

6 H₂O ---> O₂ + 4 H₃O⁺ + 4 e⁻ (2),

2 Cl⁻ ---> Cl₂ + 2 e⁻ (3).

Chlor wiederum dissoziiert in Wasser entsprechend der nachfolgenden Gleichgewichtsreaktion (4) in Hypochloritionen (OCl⁻) und Chloridionen (Cl⁻), welche sich wiederum mit einem geeigneten Kation, z.B. Na⁺ aus dem Elektrolyt, oder mit einem Proton bzw. einem H₃O⁺-Ion zu dem entsprechenden (Natrium)salz bzw. zu der entsprechenden Säure, d.h. zu hypochloriger Säure (HClO) bzw. - nach Zusammenlagerung mit in der Wasser-/Elektrolytlösung vorhandenen Kationen - Hypochloriten und Chlorwasserstoff bzw. verdünnter Salzsäure (HCl) reagieren können:

Cl₂ + 3 H₂O <===> 2 H₃O⁺ + OCl⁻ + Cl⁻ (4).

Bei einer geeigneten Steuerung des pH-Wertes und des Redoxpotentials, wie es in der oben erwähnten WO 2007/093385 A1 detailliert erläutert ist, läßt sich das Gleichgewicht zugunsten der am meisten erwünschten Komponente hypochlorige Säure verschieben, wodurch ein bereits in sehr geringen Konzentrationen äußerst wirksames Desinfektionsmittel erhalten werden kann. Ferner können aus den vorgenannten, an der Anode gebildeten Stoffen durch Sekundärreaktionen in geringeren Mengen weitere Stoffe erzeugt werden, welche ebenfalls bekanntermaßen im Hinblick auf eine Desinfektion von Wasser wirksam sind. Hierbei handelt es sich insbesondere um Wasserstoffperoxid (H₂O₂, Reaktionsgleichung (5)), Ozon (O₃, Reaktionsgleichung (6)), Chlordioxid (ClO₂, Reaktionsgleichung (7)), Chlorate (ClO₃⁻, Reaktionsgleichung (8)) und verschiedene Radikale (Reaktionsgleichungen (9) und (10)).

4 H₂O ---> H₂O₂ + 2 H₃O⁺ + 2 e⁻ (5),

O₂ + 3 H₂O ---> O₃ + 2 H₃O⁺ + 2 e⁻ (6),

Cl⁻ + 4 OH⁻ ---> ClO₂ + 2 H₂O + 5 e⁻ (7),

3 OCl⁻ ---> ClO₃⁻ + 2 Cl⁻ (8),

5 H₂O ---> HO₂⁻ + 3 H₃O⁺ + 3 e⁻ (9),

H₂O₂ + H₂O ---> HO₂⁻ + H₃O⁺ + e⁻ (10).

Ein Nachteil von mittels elektrochemischer Aktivierung hergestellten Desinfektionsmitteln besteht darin, daß sie in aller Regel eine nur begrenzte Haltbarkeit bzw. Lagerfähigkeit besitzen. Zwar läßt sich, wie bereits angedeutet, die Haltbarkeit des Desinfektionsmittels durch geeignete Steuerung bzw. Regelung, wie insbesondere einer solchen gemäß der oben erwähnten WO 2007/093385 Al, auf eine Dauer von etwa einem halben Jahr bis ein Jahr erhöhen, doch sollte das Desinfektionsmittel in diesem Fall insbesondere in einem geschlossenen Behälter aufbewahrt und in geeigneter Weise gekühlt werden. Vornehmlich im Falle einer relativ großen Oberfläche des Desinfektionsmittels mit Umgebungsluft läßt sich eine relativ schnell eintretende Neutralisierung des Desinfektionsmittels indes praktisch nicht verhindern. Dies liegt darin begründet, daß gemäß der obigen Reaktionsgleichung (4) ein Gleichgewicht zwischen gelöstem Chlor (Cl₂) und hypochloriger Säure (HOCl) bzw. deren Salzen herrscht, wobei das Chlor aus der Lösung ausgast und sich somit der Anteil von Chlor sowie insbesondere der desinfektiös wirksamen hypochlorigen Säure (Hypochlorsäure) bzw. auch deren Salze, den Hypochloriten, fortwährend verringert, weil sich das Gleichgewicht zuungunsten der OCl⁻-Ionen verschiebt, welche zu Chlorgas reagieren, das sich wiederum aus der Lösung verflüchtigt. Darüber hinaus neigt hypochlorige Säure insbesondere bei steigendem pH-Wert zur Abspaltung ihres Protons unter Bildung des entsprechenden Hypochlorits, welches, wie oben erwähnt, gegenüber undissoziierter hypochloriger Säure nur vermindert desinfektiös wirksam ist.

Die US 4 561 994 A beschreibt eine stabile, tensidfreie, wäßrige Hypochloritpaste, welche in Wasser dispergierbar ist. Um die pastenförmige Konsistenz zu erzielen, wird ein Verdickungsmittel aus der Gruppe der nicht reaktiven anorganischen Substanzen, ausgenommen Alkalimetallsilikate, in einem Anteil zwischen 5 und 70% eingesetzt, wobei unter anderem ein Verdickungsmittel auf der Basis von amorphen Silicagelen erwähnt ist. Der pH-Wert der Hypochloritpaste ist stark basisch.

Die WO 01/28336 A1 beschreibt eine desinfektiös wirksame Hydrogelzubereitung mit einem elektrolytischen Chloroxidationsmittel, welches Hypochloritionen und Hypochlorsäure enthält und einen aktiven Chlorgehalt von 100 bis 11.000 ppm aufweist, einem Verdickungsmittel, einem Elektrolyt und Wasser, wobei die Hydrogelzubereitung zur Desinfektion von organischem Gewebe dienen kann. Während es sich bei dem Elektrolyt z.B. um Kochsalz handeln kann, kommen als Verdickungsmittel unter anderem kristalline Siliciumverbindungen in Form von Schichtstrukturen auf der Basis von natürlicher Tonerde, wie Laponit, in Betracht. Die Verdickungsmittel sorgen für eine Viskosität von einer dickflüssigen Flüssigkeit bis hin zu einem dicken, leicht trüben Gel.

In der WO 01/00030 A1 geht es um ein Verfahren zur Herstellung, Speicherung und Messung eines Desinfektionsmittels sowie um einen speziellen Druckbehälter zur Aufnahme des Desinfektionsmittels. Als Ausführungsbeispiel für ein mögliches Desinfektionsmittel ist unter anderem ein solches angegeben, welches Borax, Seifenpulver, Siliciumdioxid und eine Natriumhypochloritlösung bei einem stark basischen pH-Wert enthält.

In der US 4 287 079 A geht es um ein weiteres Desinfektionsmittel auf wäßriger Basis, welches neben Natriumhypochlorit ein Verdickungsmittel enthält, das aus einer Mischung aus Smektitton und pyrogenen Kieselsäuren, wie einem Produkt mit dem Handelsnamen "CAB-O-SIL.M5" (Cabot Corp.), gebildet ist. Der pH-Wert des Desinfektionsmittels beträgt zwischen 10 und 12, um das als Bleichmittel verwendete Natriumhypochlorit zu stabilisieren.

Den JP 2005-150 216 A und JP 2006-012 980 A sind Schleifflüssigkeiten zur Polierung von Halbleiterplatten zu entnehmen, welche aus einer wäßrigen Lösung hypochloriger Säure mit hierein eindispergierten, feinpartikulären Silicapartikeln bestehen, wobei letztere zur Erzielung der abrasiven Wirkung der Schleifflüssigkeit dienen.

In dem in "Analytical Sciences", August 1998, Vol 14 der Japanischen Gesellschaft für Analytische Chemie erschienenen Artikel "Spectroscopic Characterization and the pH Dependence of Bactericidal Activity of the Aqueous Chlorine Solution" (Nakagawara, S. et al.) ist schließlich allgemein die Abhängigkeit der bakteriellen Aktivität in chlorhaltigen Lösungen, welche mittels elektrochemischer Aktivierung erhalten worden sind, von deren pH-Wert beschrieben. Als Ergebnis wurde dort - was die Spezies *E. coli* und *Bacillus subtilis* betrifft - ein Maximum der bakteriellen Aktivität in solchen Lösungen bei einem pH-Wert zwischen 4 und 5 festgestellt.

Der Erfindung liegt die Aufgabe zugrunde, eine zur Verwendung Desinfektionsmittel dienende wäßrige, hypochlorige Säure (HOCl) enthaltende Lösung dahingehend weiterzubilden, daß ihre Lebensdauer, insbesondere auch im Falle einer verhältnismäßig großen Oberfläche derselben mit der Umgebung, erhöht wird.

Erfindungsgemäß wird diese Aufgabe bei der Verwendung einer wäßrigen, hypochlorigen Säure enthaltenden Lösung als Desinfektionsmittel dadurch gelöst, daß die Lösung einen pH-Wert zwischen 2,5 und 8 aufweist und ferner einen Anteil an amorphem Siliciumdioxid (Si0₂) enthält.

Die Herstellung einer solchen Lösung kann dadurch erfolgen, daß der als Desinfektionsmittel verwendeten wäßrigen, hypochlorige Säure (HOCl) enthaltenden Lösung mit einem pH-Wert von 2,5 bis 8 amorphes Siliciumdioxid (SiO₂ zugesetzt wird.

Überraschenderweise wurde gefunden, daß sich die Haltbarkeit des Desinfektionsmittel - bzw. insbesondere die Lebensdauer der zur Desinfektion erwünschten, auch als Hypochlorsäure bezeichneten hypochlorigen Säure (HOCl) - durch die Zugabe von amorphem Siliciumdioxid deutlich erhöhen läßt, wobei vermutet wird, daß amorphes Siliciumdioxid die Gleichgewichtsreaktion gemäß der obigen Reaktionsgleichung (4) hemmt oder die Hypochloritionen - sei es in Form von hypochloriger Säure oder in Form von Hypochloriten - fixiert, so daß die Hypochloritionen in der Lösung verbleiben und dort ihrer desinfizierenden Funktion nachkommen können. Insbesondere wurde überraschenderweise gefunden, daß amorphes Siliciumdioxid eine Abspaltung des Protons von hypochloriger Säure zu hemmen vermag, so daß diese nicht zu den weniger desinfektiös wirksamen Hypochloritionen dissoziiert. Dies überrascht insoweit, als nicht amorphes, d.h. kristallines Siliciumdioxid bekanntermaßen als Ionentauscher wirkt und die Abspaltung des Protons von hypochloriger Säure zu dem Hpypochlorition (OCl⁻), welches sich dann mit einem geeigneten Kation zu dem entsprechenden Salz zusammenlagert, begünstigt. Darüber hinaus wird gleichfalls auch ein andernfalls gegebenenfalls auftretender, leichter Geruch nach Chlor, welcher in der überwiegenden Anzahl aller Anwendungsfälle unerwünscht ist, vermieden, da das in dem Desinfektionsmittel gelöste Chlorgas in Lösung (im Gleichgewicht mit hypochloriger Säure oder auch mit den Hypochloritionen) verbleibt und nicht durch Reaktion der Hypochloritionen bzw. vor allem der hypochlorigen Säure zu Chlorgas ständig "nachgeliefert" wird. Ein weiterer Vorteil des erfindungsgemäßen Desinfektionsmittels besteht auch darin, daß Siliciumdioxid in amorpher Form Reste von (abgetöteten) Bakterien, Viren oder anderer Mikroben anzulagern vermag, welche nach der Desinfektionsbehandlung anläßlich des Entfernens des Desinfektionsmittels, z.B. durch Abspülen, problemlos mit entfernt werden können.

Das amorphe, d.h. nicht kristalline Siliciumdioxid kann beispielsweise in Form von amorphen Kieselsäuren und/oder amorphen Kieselsäureanhydriden vorliegen. Dabei sind mit "amorphen Kieselsäuren" im Sinne der Erfindung sowohl amorphe Polykieselsäuren der allgemeinen Formel [-Si(OH)₂-O-] als auch amorphe Ortho- (Si(OH)₄) und Metakieselsäuren ((-SiO-O-]) sowie sämtliche Mischformen derselben angesprochen. Was die "amorphen Kieselsäureanhydride" anbelangt, so umfassen diese sowohl amorphe Kieselgele und amorphe gefällte Kieselsäuren (SiO₂), wie sie beispielsweise durch Reaktion von Wasserglas (Na₂SiO₃) mit Schwefelsäure unter Entstehung von Natriumsulfat erhältlich sind, als auch amorphe pyrogene Kieselsäuren, wie sie z.B. durch Reaktion von Siliciumtetrachlorid (SiCl₄) mit Wasser unter Entstehung von Chlorwasserstoff, erhältlich sind und in der Regel in Form feiner Pulver vorliegen. Ferner kann unmittelbar Kieselerde in amorpher Form eingesetzt werden, welche zweckmäßig zu wenigsten 90 Mass.-% SiO₂ aufweist.

Gemäß einer bevorzugten Ausführungsform des erfindungsgemäßen Desinfektionsmittels ist vorgesehen, daß es einen hinreichenden Anteil an amorphem Siliciumdioxid enthält, um die Viskosität der wäßrigen, hypochlorige Säure enthaltenden Lösung zu erhöhen, wobei das Desinfektionsmittel insbesondere eine mehr oder minder gelartige Konsistenz aufweisen kann. Die Herstellung eines solchen Desinfektionsmittels geschieht folglich derart, daß das amorphe Siliciumdioxid der wäßrigen, hypochlorige Säure enthaltenden Lösung in einem hinreichenden Anteil zugesetzt wird, um ihre Viskosität zu erhöhen, wobei insbesondere ein hinreichender Anteil an amorphem Siliciumdioxid zugesetzt werden kann, um die Lösung zu gelieren. In jedem Fall wird das amorphe Siliciumdioxid zweckmäßig möglichst homogen in die wäßrige, hypochlorige Säure enthaltende Lösung eindispergiert, was mit praktisch beliebigen geeigneten Rührern, Homogenisatoren etc. geschehen kann.

Ein solches Desinfektionsmittel weist den weiteren Vorteil einer äußerst einfachen Anwendung z.B. für die Desinfektion von geneigten Oberflächen oder auch von menschlicher oder tierischer Haut auf, da das Desinfektionsmittel nicht infolge Schwerkraft von seinem ihm zugedachten Wirkungsort entfernt wird, sondern dort immobilisierbar ist, wobei es gleichwohl seine hervorragenden Desinfektionseigenschaften behält, welche zudem sehr gut hautverträglich sind. Folglich wird sowohl die Einwirkungsdauer als auch die Haltbarkeit des Desinfektionsmittels am Wirkungsort erhöht.

Das Massenverhältnis der wäßrigen, hypochlorige Säure enthaltenden Lösung zu dem amorphen Siliciumdioxid kann zweckmäßig zwischen etwa 100:1 und etwa 1:1, insbesondere zwischen etwa 50:1 und etwa 2:1, betragen und richtet sich selbstverständlich auch nach der jeweils gewünschten Viskosität des Produktes. Das amorphe Siliciumdioxid wird der wäßrigen, hypochlorige Säure enthaltenden Lösung dann in dem gewünschten Massenverhältnis zugesetzt und, wie bereits erwähnt, dort möglichst homogen verteilt.

Während das Desinfektionsmittel auf Basis der wäßrigen, hypochlorige Säure enthaltenden Lösung grundsätzlich auf beliebige Weise erhalten werden kann, sieht eine bevorzugte Ausführungsform des erfindungsgemäßen Desinfektionsmittels vor, daß es von einer elektrochemisch aktivierten, verdünnten Wasser-/Elektrolytlösung gebildet ist, d.h. die wäßrige, hypochlorige Säure enthaltende Lösung wird durch elektrochemische Aktivierung (ECA) einer verdünnten Wasser-/ Elektrolytlösung erzeugt. Dies kann insbesondere dadurch geschehen, indem Wasser, z.B. Leitungswasser oder auch demineralisiertes Wasser, gemäß dem in der oben zitierten WO 2007/093385 A1 beschriebenen Verfahren elektrochemisch behandelt wird. Eine weiterhin vorteilhafte Ausgestaltung sieht in diesem Zusammenhang insbesondere vor, daß das Desinfektionsmittel ausschließlich elektrochemisch aktivierte, anodische, verdünnte Wasser-/Elektrolytlösung enthält, d.h. es wird lediglich die an der positiven Elektrode (Anode) erhaltene, elektrochemisch aktivierte, verdünnte Wasser-/ Elektrolytlösung zur Herstellung des Desinfektionsmittels unter Eindispergieren von amorphem Siliciumdioxid verwendet.

Die Herstellung einer solchen anodischen Lösung kann hierbei, wie als solches in der oben zitierten WO 2007/093385 A1 erläutert, derart erfolgen, daß die elektrochemisch aktivierte, verdünnte Wasser-/Elektrolytlösung dadurch erhalten wird, indem Wasser eine Elektrolytlösung, insbesondere eine Natrium- und/oder Kaliumchloridlösung, zugesetzt und das mit der Elektrolytlösung beaufschlagte Wasser in Form einer verdünnten Wasser-/Elektrolytlösung in einem Elektrolysereaktor mit wenigstens einem Kathodenraum mit einer Kathode und mit wenigstens einem von dem Kathodenraum räumlich, insbesondere mittels eines Diaphragmas oder einer Membran, getrennten Anodenraum mit einer Anode durch Anlegen einer Gleichspannung an die Elektroden mit einem elektrischen Strom beaufschlagt wird, um die verdünnte Wasser-/ Elektrolytlösung in einen zur Desinfektion geeigneten, metastabilen Zustand zu versetzen, in welchem sie einen hohen Anteil an hypochloriger Säure enthält. Sofern nur die elektrochemisch aktivierte, anodische Wasser-/Elektrolytlösung zur Herstellung des erfindungsgemäßen Desinfektionsmittels verwendet werden soll, so wird ausschließlich die auch als "Anolyt" bezeichnete, in dem Anodenraum des Reaktors behandelte Lösung, d.h. die aus dem Anodenraum des Elektrolysereaktors austretende Lösung, eingesetzt, während die in dem Kathodenraum behandelte, auch als "Katholyt" bezeichnete kathodische Lösung verworfen werden kann.

Das erfindungsgemäße Desinfektionsmittel kann je nach Anwendung zweckmäßig einen Summenparameter freien Chlors zwischen etwa 10 mg/l und etwa 70 mg/l, insbesondere zwischen etwa 20 mg/l und etwa 60 mg/l, vorzugsweise zwischen etwa 30 mg/l und etwa 50 mg/l, aufweisen. Ein solcher Wert wird vorzugsweise bereits anläßlich der Erzeugung, z.B. durch elektrochemische Aktivierung, der wäßrigen, hypochlorige Säure enthaltenden Lösung selbst eingestellt, wobei selbstverständlich in Abhängigkeit des gewünschten Endproduktes auch demgegenüber niedrigere oder höhere Werte möglich sind. Eine Einstellung höherer Werte kann z.B. auch dann sinnvoll sein, wenn das Desinfektionsmittels weitere Stoffe enthalten soll, so daß der Summenparameter an freiem Chlor in der Lösung unter Berücksichtigung von Verdünnungseffekten derart eingestellt wird, daß das Endprodukt einen solchen Wert besitzt.

Das erfindungsgemäße Desinfektionsmittel kann je nach Verwendung einen pH-Wert zwischen etwa 2,5 und etwa 8, insbesondere bis etwa 7, sowie ein Redoxpotential zwischen etwa 1100 mV und etwa 1360 mV, insbesondere zwischen etwa 1150 mV und etwa 1360 mV, vorzugsweise zwischen etwa 1200 mV und etwa 1360 mV, aufweisen, wobei sich - was die wäßrige, hypochlorige Säure enthaltende Lösung, z.B. in Form einer elektrochemisch aktivierten, anodischen, verdünnten Wasser-/Elektrolytlösung, als sol-che betrifft - insbesondere ein pH-Wert im Bereich von 2,5 bis 3,5, vorzugsweise von 2,8 bis 3,2 und insbesondere im Bereich von 3, sowie ein Redoxpotential im Bereich von 1240 mV und etwa 1360 mV, vorzugsweise zwischen etwa 1280 mV und etwa 1360 mV, insbesondere zwischen etwa 1320 mV und etwa 1360 mV, z.B. von etwa 1340 mV, als vorteilhaft erwiesen hat, um für einen möglichst hohen Gehalt der Lösung an hypochloriger Säure bei einem nur niedrigen Gehalt an Cl₂ zu sorgen, welches leicht aus der Lösung ausgast und einen unerwünschten stechenden Geruch verursachen kann.

Hinsichtlich der Herstellung des Desinfektionsmittels kann aus diesem Grund vorgesehen sein, daß der pH-Wert der wäßrigen, hypochlorige Säure enthaltenden Lösung, z.B. in Form einer elektrochemisch aktivierten, anodischen Wasser-/Elektrolytlösung, anläßlich der elektrochemischen Aktivierung auf einen Wert zwischen 2,5 und 3,5, insbesondere zwischen 2,7 und 3,3, vorzugsweise zwischen 2,8 und 3,2, eingestellt wird, wobei ferner vorzugsweise das Redoxpotential der elektrochemisch aktivierten, anodischen Wasser-/Elektrolytlösung auf einen Wert zwischen 1240 mV und 1360 mV, insbesondere zwischen 1280 mV und 1360 mV, vorzugsweise zwischen 1320 mV und 1360 mV, eingestellt wird. Die Verfahrensführung der elektrochemischen Aktivierung zum Erhalt eines solchen Desinfektionsmittels kann folglich derart gesteuert werden, daß die aus dem Anodenraum des Elektrolysereaktors, welcher von dem Kathodenraum durch ein(e) elektrisch leitfähige(s) Diaphragma/Membran räumlich getrennt ist, austretende Lösung - bzw. die elektrochemisch aktivierte, anodische, verdünnte Wasser-/Elektrolytlösung (Anolyt) -, einen pH-Wert bzw. ein Redoxpotential in dem genannten Bereich aufweist, d.h. die Steuerung des pH-Wertes bzw. des Redoxpotentials geschieht derart, daß sich deren genannten Werte am Ende des Reaktors in dessen Anodenraum eingestellt haben. Das Redoxpotential bezieht sich in diesem Zusammenhang stets auf die Normal- (NHE) bzw. Standardwasserstoffelektrode (SHE), d.h. vs. NHE bzw. vs. SHE. Verschiedene Möglichkeiten einer Steuerung der elektrochemischen Aktivierung, welche zu einem solchen Desinfektionsmittel führen, sind in der oben erwähnten WO 2007/093385 A1 detailliert beschrieben. Die für das erfindungsgemäße Desinfektionsmittel vorzugsweise verwendete elektrochemisch aktivierte, anodische, verdünnte Wasser-/Elektrolytlösung mit den genannten pH- und/oder Redoxpotentialwerten hat sich insoweit als außerordentlich vorteilhaft erwiesen, als es nicht nur eine praktisch gleichbleibende Desinfektionswirkung - insbesondere auch im Falle eines in Wasser verdünnten Einsatzes - sicherstellt, sondern auch für eine hinreichende Depotwirkung sorgt, welche auch im Falle von starken mikrobiellen Verunreinigungen anhält. Überdies läßt sich, wie bereits angedeutet, die Entstehung von Chlorgas gemäß der obigen Reaktionsgleichung (3) auf ein Minimum begrenzen, so daß die für das erfindungsgemäße Desinfektionsmittel vorzugsweise eingesetzte elektrochemisch aktivierte, anodische, verdünnte Wasser-/Elektrolytlösung allenfalls einen sehr schwachen, in aller Regel gar keinen Chlorgeruch besitzt. Die Lösung beinhaltet größtenteils hypochlorige Säure (HOCl) sowie gegebenenfalls zusätzlich geringe Mengen an Hypochloriten, wie Natriumhypochlorit (NaClO) und metastabile radikalische Verbindungen sowie in ebenfalls geringeren Mengen Chlorwasserstoff anstelle von Chlorgas (Cl₂), d.h. das Gleichgewicht der obigen Reaktionsgleichung (4) ist bei dem genannten pH- und/oder Redoxpotentialwertebereich offenbar nach rechts verschoben.

Soll nun der pH-Wert des erfindungsgemäßen Desinfektionsmittels, d.h. zumindest der wäßrigen, hypochlorige Säure enthaltenden Lösung mit dem dieser zugesetzten amorphem Siliciumdioxid, demgegenüber erhöht sein, um ihn z.B. auf einen hautfreundlichen pH-Wert im Bereich von etwa 4 bis etwa 8, vorzugsweise im Bereich von bis zu etwa 7, insbesondere bis zu etwa 6, beispielsweise bis zu etwa 5,5 oder bis zu etwa 5, anzuheben und das Desinfektionsmittel somit einer medizinisch wirksamen Behandlung, z.B. zur Desinfektion von Wunden, zugänglich zu machen, so kann der pH-Wert des erfindungsgemäßen Desinfektionsmittels bedarfsweise durch Zugabe eines Puffers, insbesondere auf einen Wert von bis zu etwa 8, vorzugsweise auf einen Wert von bis zu etwa 7 oder bis zu den vorstehen genannten Werten, erhöht werden. Hierbei kann zwar das erwähnte Gleichgewicht zwischen der hypochlorigen Säure oder auch ihrer OCl⁻-Ionen und Cl₂ zuungunsten erstgenannter verschoben werden, doch wirkt in diesem Fall der erfindungsgemäße Zusatz des amorphen Siliciumdioxids wiederum einer solchen Verschiebung entgegen, so daß auch bei solchen, verhältnismäßig hohen pH-Werten eine einwandfreie Desinfektionswirkung erzielt werden kann und ferner selbst bei einem pH-Wert bis etwa 8,0 das Redoxpotential eines aus der Lösung mit amorphem SiO₂ bestehenden Desinfektionsmittels stets deutlich oberhalb 1100 mV stabil bleibt und das hierin enthaltene freie Chlor vornehmlich in Form von hypochloriger Säure vorliegt.

Als Puffer kommen grundsätzlich praktisch beliebige bekannte Puffer in Betracht, wobei sich z.B. ein solcher auf Carbonat-/Hydrogencarbonatbasis bewährt hat, welcher zudem gesundheitlich völlig unbedenklich ist. Überdies kann bereits das amorphe Siliciumdioxid selbst eine gewisse Pufferwirkung besitzen, so daß allein durch den Zusatz von amorphem SiO₂ je nach Zusammensetzung des für die elektrochemisch aktivierte Lösung eingesetzten Wassers eine gewisse Erhöhung deren pH-Wertes bis in einen Bereich von etwa 5 bis 5,5 erzielt werden kann.

Darüber hinaus kann es von Vorteil sein, wenn zur Herstellung der wäßrigen, hypochlorige Säure enthaltenden Lösung in Form einer elektrochemisch aktivierten, verdünnten Wasser-/Elektrolytlösung Reinwasser eingesetzt wird, um für reproduzierbare Herstellungsbedingungen zu sorgen und insbesondere den Einfluß von gegebenenfalls in dem Rohwasser enthaltenen Ionen auf die elektrochemische Aktivierung zu minimieren. Hierzu kann das zur Herstellung der elektrochemisch aktivierten, verdünnten Wasser-/Elektrolytlösung eingesetzte Rohwasser insbesondere zunächst einem Membranverfahren, wie Umkehrosmose, Mikro-, Nano- oder Ultrafiltration, unterzogen werden. Auf diese Weise kann insbesondere die spezifische elektrische Leitfähigkeit des elektrochemisch zu aktivierenden Wassers - bzw. genauer: dessen spezifische ionische Leitfähigkeit, welche auf der Leitfähigkeit des Wassers bzw. der Wasser-/Elektrolytlösung aufgrund der hierin gelösten, bewegungsfähigen Ionen beruht - sowie dessen Härte und gegebenenfalls auch die Konzentration hierin enthaltener organischer Inhaltsstoffe abgesenkt werden, wobei sich ein Maximalwert der spezifischen Leitfähigkeit von etwa 350 µS/cm, vorzugsweise zwischen etwa 0,055 µS/cm und etwa 150 µS/cm und insbesondere zwischen etwa 0,055 µS/cm und etwa 100 µS/cm, vor dem Zusetzen der Elektrolytlösung (was die Leitfähigkeit des eingesetzten Wassers in aller Regel ohnehin um ein Vielfaches erhöht), als vorteilhaft erwiesen hat. Auf diese Weise wird anläßlich der Erzeugung der für das erfindungsgemäße Desinfektionsmittel verwendeten elektrochemisch aktivierten, verdünnten Wasser-/Elektrolytlösung eine noch bessere Reproduzierbarkeit hinsichtlich seiner Desinfektions- und Depotwirkung erzielen, und zwar praktisch unabhängig von dem eingesetzten Wasser. Darüber hinaus lassen sich in dem elektrochemisch zu aktivierenden Wasser gegebenenfalls enthaltene Ionen, welche bei der elektrochemischen Aktivierung - wenn auch in nur geringen Konzentrationen - in gesundheitlich bedenkliche Stoffe umgewandelt werden, zumindest weitestgehend eliminieren. Als Beispiel seien Bromidionen erwähnt, welche zu Bromat oxidiert werden können, welches in höheren Konzentrationen eine cancerogene Wirkung besitzt.

Wie bereits erwähnt, ist das erfindungsgemäße Desinfektionsmittels insbesondere zur Desinfektion von Oberflächen einschließlich menschlicher und tierischer Haut geeignet, wobei ihm das der wäßrigen, hypochlorige Säure enthaltenden Lösung zugesetzte amorphe Siliciumdioxid eine lange Desinfektionswirkung sowie, sofern gewünscht, eine hinreichend hohe Viskosität verleiht, um es nach Art eines Gels auf die jeweilige Oberfläche auftragen zu können. Insbesondere auch im Falle einer Anwendung des Desinfektionsmittels zur Herstellung eines Arzneimittels Desinfektion von menschlicher oder tierischer Haut einschließlich Schleimhaut, wie zur Desinfektion von Wunden, kommen ihm zusätzlich die folgenden Eigenschaften zugute: Aufgrund seines hohen Wassergehaltes wird die Wunde vor Austrocknen geschützt und die Zellteilung und Zellwanderung begünstigt. Es verhindert zuverlässig Infektionen, wobei seine Bestandteile äußerst hautverträglich und gesundheitlich unbedenklich sind, so daß es beispielsweise auch im Mund- und Rachenraum eingesetzt werden kann. Dies gilt insbesondere auch für das amorphe Siliciumdioxid, welches im Gegensatz zu den meisten kristallinen Siliciumdioxidmodifikationen weitestgehend inert ist und keine für den Organismus potentiell schädlichen Ionentauscheigenschaften besitzt. Es enthält kein Jod, ist farbneutral und vermag überschüssiges Wundsekret aufzunehmen. Es ist problemlos mit Wasser abspülbar und kann bedenkenlos über den Hausmüll oder die Kanalisation entsorgt werden.

Darüber hinaus ist das erfindungsgemäße Desinfektionsmittels selbstverständlich auch zur Desinfektion von flüssigen Medien gleich welcher Art, wie insbesondere auch Wasser, mit welchem es praktisch unbegrenzt mischbar ist, geeignet, wobei es dem jeweiligen Medium problemlos in der gewünschten Menge zudosiert werden kann. Das amorphe Siliciumdioxid kann in diesem Fall aufgrund seiner adsorptiven Eigenschaften zusätzlich nach Art eines Fällungsmittels wirken, an welches sich in dem zu desinfizierenden Medium vorhandene Schwebstoffe anlagern können.

Weitere Merkmale und Vorteile der Erfindung ergeben sich aus der nachfolgenden Beschreibung eines Ausführungsbeispiels eines Verfahrens zur Herstellung eines erfindungsgemäßen Desinfektionsmittels unter Bezugnahme auf die Zeichnung. Dabei zeigen:
- Fig. 1: ein schematisches Fließbild einer Ausführungsform eines Verfahrens zur Herstellung eines Desinfektionsmittels auf der Basis einer wäßrigen, hypochlorige Säure enthaltenden Lösung in Form einer elektrochemisch aktivierten, verdünnten Wasser-/ Elektrolytlösung mit in diese eindispergiertem, amorphem Siliciumdioxid;
- Fig. 2: eine geschnitten dargestellte Detailansicht des Elektrolysereaktors gemäß Fig. 1; und
- Fig. 3: eine geschnitten dargestellte Detailansicht des Mischers gemäß Fig. 1.

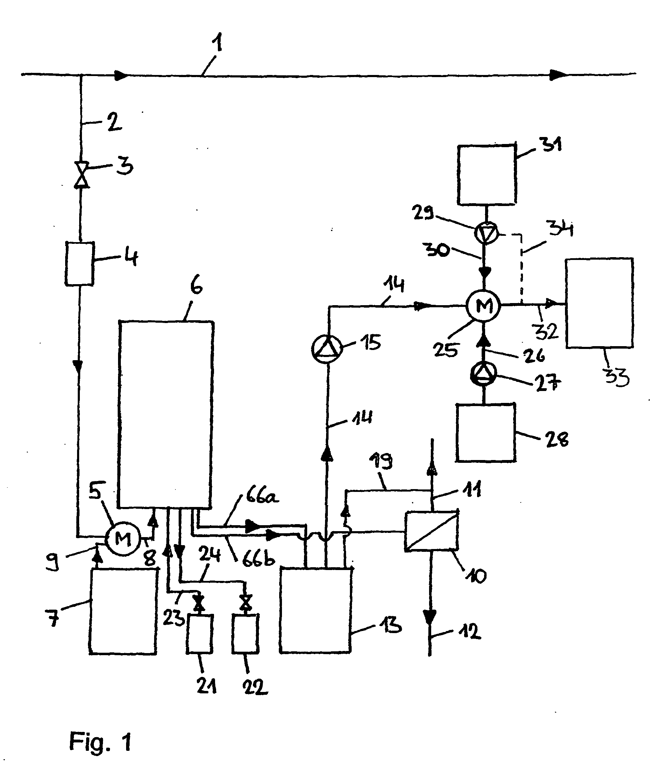

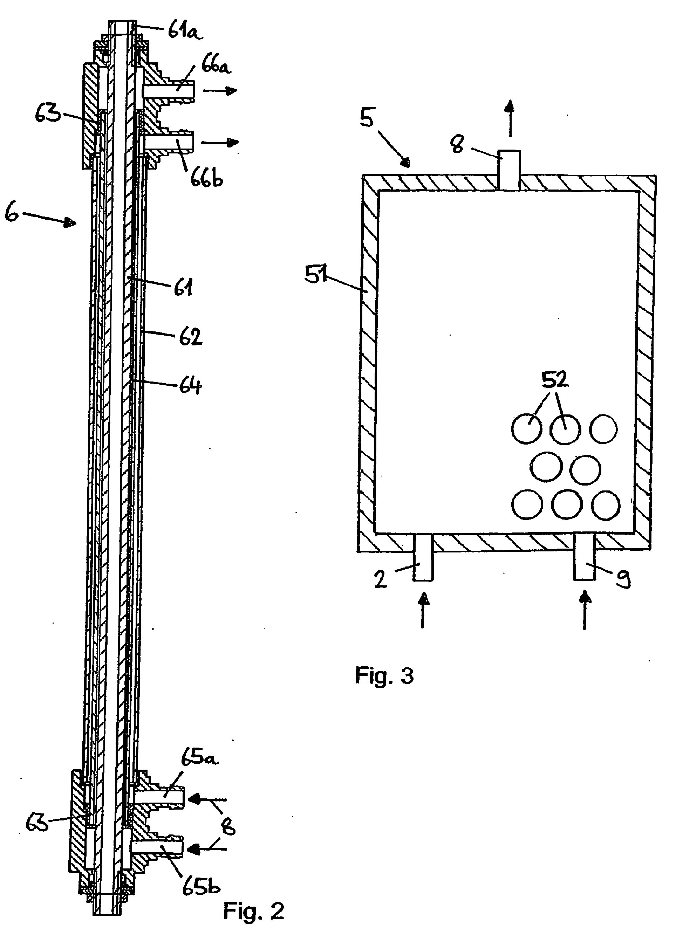

Die in Fig. 1 schematisch dargestellte, zur kontinuierlichen oder semikontinuierlichen Durchführung eines Verfahrens zur Herstellung eines erfindungsgemäßen Desinfektionsmittels geeignete Vorrichtung zweigt aus einer Hauptwasserleitung 1 über eine Abzweigleitung 2 Wasser ab, welches als Rohwasser für die elektrochemische Aktivierung (ECA) verwendet wird. Die Hauptwasserleitung 1 kann beispielsweise Teil eines öffentlichen Wasserversorgungssystems sein. Die Abzweigleitung 2 ist mit einem Ventil 3, insbesondere in Form eines Steuerventils, sowie mit einem Filter 4, insbesondere in Form eines Feinfilters mit einer Lochweite von beispielsweise etwa 80 bis 100 µm, ausgestattet und mündet über einen weiter unten unter Bezugnahme auf Fig. 3 näher erläuterten Mischer 5 in einen ebenfalls weiter unten unter Bezugnahme auf Fig. 2 näher beschriebenen Elektrolysereaktor 6. Über die Abzweigleitung 2 ist somit ein mittels des Steuerventils 3 bedarfsweise steuerbarer Teilstrom des in der Hauptwasserleitung 1 geförderten Wassers in den Elektrolysereaktor 6 überführbar.

Der Mischer 5 steht zulaufseitig einerseits mit der Abzweigleitung 2, andererseits mit einem Reservoir 7 zur Aufnahme einer Elektrolytlösung - hier z.B. einer im wesentlichen gesättigten Natrium- und/oder Kaliumchloridlösung - in Verbindung, welche in dem Mischer 5 möglichst homogen miteinander vermischt werden und über eine gemeinsame, ablaufseitige Leitung 8 des Mischers 5 in den Elektrolysereaktor 6 gelangen. Die von dem Reservoir 7 in den Mischer 5 führende Leitung 9 ist ferner mit einer in Fig. 1 nicht dargestellten Dosierpumpe ausgestattet, um dem in der Abzweigleitung 2 geförderten Wasser eine definierte Menge an Elektrolytlösung zuzusetzen. Wie insbesondere aus Fig. 3 ersichtlich, ist der Mischer 5 beim vorliegenden Ausführungsbeispiel von einem Kugelmischer gebildet, welcher eine konstant gleichmäßige Durchmischung des Wassers mit der Elektrolytlösung sicherstellt. Er umfaßt im wesentlichen einen etwa zylindrischen Behälter 51, an dessen entgegengesetzten Enden die Zuläufe 2, 9 bzw. der Ablauf 8 angeschlossen sind und in welchem eine Schüttung aus in Fig. 3 exemplarisch angedeuteten Kugeln 52 oder anderem Schüttgut angeordnet ist, durch welches das Wasser und die Elektrolytlösung hindurch fließen, wobei die Kugeln 52 zu Schwingungen angeregt werden und dabei eine sehr homogene Durchmischung des Wassers mit der diesem zugesetzten Elektrolytlösung gewährleisten.

Wie insbesondere der Fig. 2 zu entnehmen ist, umfaßt der Elektrolysereaktor 6 eine Anode 61, welche beim vorliegenden Ausführungsbeispiel z.B. von einem mit katalytisch wirksamem Rutheniumdioxid (RuO₂) beschichteten Hohlrohr aus Titan gebildet ist und an welches endseitig über ein Außengewinde 61a der Pluspol einer nicht näher dargestellten Spannungsquelle anschließbar ist. Alternativ oder zusätzlich zu Rutheniumoxid kann beispielsweise auch eine Beschichtung auf der Basis von Iridiumdioxid (IrO₂) oder einer Mischung beider (RuO_{2/}IrO₂) oder anderer Oxide, wie Titandioxid (TiO₂), Bleidioxid (PbO₂) und/oder Mangandioxid (MnO₂), vorgesehen sein. Der Elektrolysereaktor 6 umfaßt des weiteren eine Kathode 62, welche zweckmäßig aus Edelstahl oder ähnlichen Materialien, wie Nickel (Ni), Platin (Pt), etc., gefertigt und beim vorliegenden Ausführungsbeispiel ebenfalls von einem Hohlrohr gebildet ist, innerhalb dessen die Anode 61 koaxial angeordnet ist. Die Kathode 62 ist mittels z.B. sie außenseitig umgreifender Klemmen (nicht dargestellt) an den Minuspol der nicht näher weitergegebenen Spannungsquelle anschließbar. Koaxial zu der Anode 61 sowie zu der Kathode 62 und zwischen diesen ist ein mittels Dichtringen 63 abgedichtetes, rohrförmiges Diaphragma 64 angeordnet, welches den zwischen der Anode 61 und der Kathode 62 befindlichen, ringförmigen Reaktionsraum in einen Anodenraum und in einen Kathodenraum abtrennt. Das Diaphragma 64 verhindert eine Vermischung der im Anodenraum und Kathodenraum befindlichen Flüssigkeit und läßt jedoch einen Stromfluß zu, welcher insbesondere für die Migration von Ionen keinen großen Widerstand darstellt. Das Diaphragma 64 ist beim vorliegenden Ausführungsbeispiel z.B. aus elektrisch - bzw. ionisch - leitfähigem, aber im wesentlichen flüssigkeitsdichtem, porösem Zirkoniumdioxid (ZrO₂) gebildet. Andere Materialien mit einem verhältnismäßig geringen Widerstand, wie Aluminiumoxid (Al₂O₃), Ionenaustauschmembranen, insbesondere solche auf Kunststoffbasis etc., können gleichfalls eingesetzt werden.

Der Elektrolysereaktor 6 besitzt ferner zwei Einlässe 65a, 65b, über welche die aus dem Mischer 5 über die Leitung 8 austretende, verdünnte Wasser-/Elektrolytlösung in den Reaktionsraum des Reaktors 6, d.h. in dessen Anodenraum und in dessen von diesem durch das Diaphragma 64 räumlich getrennten Kathodenraum, eingespeist wird. Ein hierfür vorgesehener, z.B. etwa T-förmiger Abzweig ist in Fig. 1 nicht gezeigt. Wie wiederum insbesondere aus Fig. 3 und überdies aus Fig. 1 ersichtlich, weist der Elektrolysereaktor 6 ferner zwei Auslässe 66a, 66b auf, über welche die Wasser-/ Elektrolytlösung nach der chemischen Aktivierung in dem Reaktor 6 aus diesem abführbar ist. Während der Auslaß 66a zum Abführen der elektrochemisch aktivierten Wasser-/Elektrolytlösung aus dem Anodenraum des Reaktors 6 - d.h. zum Abführen des sogenannten "Anolyts" - dient, dient der Auslaß 66b zum Abführen aus dem Kathodenraum - d.h. zum Abführen des sogenannten "Katholyts". Ferner kann vorgesehen sein, daß beim Anfahren des Elektrolysereaktors 6 über einen bestimmten Zeitraum auch der "Anolyt", d.h. die elektrochemisch aktivierte, anodische Wasser-/Elektrolytlösung, verworfen wird, um anfängliche Qualitätsbeeinträchtigungen, so lange der Elektrolysereaktor 6 noch nicht seinen gewünschten Betriebszustand erreicht hat, auszuschließen.

Nachfolgend sind die geometrischen Abmessungen des vorliegend eingesetzten Elektrolysereaktors 6 in Form einer Auflistung wiedergegeben:
Länge des Kathodenraumes: 18,5 cm;
Volumen des Kathodenraumes: 10 ml;
Fläche der Kathode: 92,4 cm²;
Länge des Anodenraumes: 21,0 cm;
Volumen des Anodenraumes: 7 ml;
Fläche der Anode: 52,7 cm²;
Abstand zwischen Kathode und Anode: ca. 3 mm (einschließlich Diaphragma).

Der Elektrolysereaktor 6 wird z.B. mit einem Wasserdurchsatz von 60 bis 140 1/h betrieben, wobei selbstverständlich auch größere Durchsätze möglich sind, indem größere Reaktoren und/oder mehrere, parallel geschaltete Reaktoren eingesetzt werden. Vorzugsweise fährt der Elektrolysereaktor 6 stets unter Vollast, wobei er bedarfsweise abgeschaltet werden kann und Spitzenlasten über einen weiter unten noch näher erläuterten Speichertank für die elektrochemisch aktivierte, anodische, verdünnte Wasser-/Elektrolytlösung abgefangen werden können.

Wie wiederum aus Fig. 1 ersichtlich, mündet der Auslaß 66b aus dem Kathodenraum des Elektrolysereaktors 6 in einen Gasabscheider 10, aus welchem das Abgas über eine optional vorgesehene Abgasleitung 11 abgeführt wird, während der Katholyt selbst, d.h. die aus dem Kathodenraum des Elektrolysereaktors 6 abgeführte Wasser-/Elektrolytlösung, über eine Leitung 12, z.B. in die Kanalisation eines kommunalen Abwassersystems, abgeführt wird. Der Auslaß 66a aus dem Anodenraum des Elektrolysereaktors 6 mündet in einen Speichertank 13, in welchem die für das Desinfektionsmittel eingesetzte, elektrochemisch aktivierte, verdünnte Wasser-/ Elektrolytlösung auf Vorrat gehalten werden kann und aus welchem der Anolyt über eine Leitung 14 abführbar ist, was mittels einer in der Leitung 14 angeordneten Dosierpumpe 15 geschehen kann.

Der Elektrolysereaktor 6 ist des weiteren mit einer in Fig. 1 nicht näher wiedergegebenen, steuerbaren Spannungsquelle ausgestattet, um zwischen der Anode 61 und der Kathode 62 (Fig. 2) den z.B. von einem Ampèremeter (nicht gezeigt) gemessenen, gewünschten Stromfluß zu steuern. Er weist ferner einen z.B. in dem Auslaß 66a für den Anolyt angeordneten pH-Meter (ebenfalls nicht gezeigt) auf, welcher alternativ beispielsweise auch in dem Speichertank 13 vorgesehen sein kann. Eine z.B. in den Reaktor 6 integrierte, steuerbare Pumpe (ebenfalls nicht gezeigt) dient zur steuerbaren Förderung der verdünnten Wasser-/Elektrolytlösung durch den Elektrolysereaktor hindurch, wobei die Pumpe den Volumenstrom und folglich die Verweilzeit der Wasser-/Elektrolytlösung in dem Reaktor 6 steuert. Eine ebenfalls nicht näher wiedergegebenen Steuereinrichtung, z.B. in Form einer elektronischen Datenverarbeitungseinheit, ist zur Steuerung der genannten Parameter derart eingerichtet, um in dem aus dem Anodenraum der Reaktors 2 über den Auslaß 66a austretenden Anolyt einen pH-Wert zwischen 2,5 und 3,5, vorzugsweise im Bereich von etwa 3,0, aufrechtzuerhalten, was beispielsweise mittels PID-Reglern geschehen kann. Bezüglich steuerungstechnischer Details zur Steuerung bzw. Regelung des pH-Wertes der elektrochemisch aktivierten, anodischen, verdünnten Wasser-/Elektrolytlösung (Anolyt) sei auf die weitere oben bereits erwähnte WO 2007/093385 A1 verwiesen.

Entsprechendes gilt für eine vorteilhafte Steuerung bzw. Regelung des Redoxpotentials der elektrochemisch aktivierten, anodischen, verdünnten Wasser-/Elektrolytlösung im Bereich von 1340 mV.

Zur Reinigung des Elektrolysereaktors 6 kann ferner ein Speicher 21 zur Aufnahme von Reinigungsflüssigkeit, z.B. Essigsäure oder dergleichen, sowie - optional - ein Speicher 22 zur Aufnahme von verbrauchter Reinigungsflüssigkeit vorgesehen sein, wobei eine von dem Speicher 21 in den Reaktor 6 führende Zuleitung 23 mit den Einlässen 65a, 65b des Reaktors 6 (vgl. Fig. 2) sowie eine aus dem Reaktor 6 in den Speicher 22 führende Ableitung 24 mit den Auslässen 66a, 66b des Reaktors 6 (vgl. Fig. 2) bedarfsweise koppelbar ist, um den Reaktor 6, d.h. sowohl dessen Kathodenraum als auch insbesondere dessen Anodenraum, zu spülen. Alternativ kann die Reinigungslösung insbesondere im Falle einer umweltverträglichen und biologisch abbaubaren Reinigungsflüssigkeit, wie Essigsäure, auch direkt in ein z.B. kommunales Abwassersystem eingespeist werden.

Um die Lebensdauer des Elektrolysereaktors 6 zu erhöhen bzw. um dessen Wartungsintervalle zu verlängern, kann diesem ferner ein in Fig. 1 nicht dargestellter Enthärter vorgeschaltet sein, welcher die Härte des Wassers z.B. auf einem Wert von höchstens 4°dH (entsprechend einer Konzentration an Erdalkalimetallionen von 0,716 mMol/1), vorzugsweise von höchstens 2°dH (entsprechend einer Konzentration an Erdalkalimetallionen von 0,358 mMol/1) hält. Der Enthärter kann herkömmlicher Bauart und z.B. mit einem geeigneten Ionenaustauscherharz bestückt sein, um die in dem Wasser gegebenenfalls enthaltenen zweiwertigen Härtebildner Calcium- und Magnesiumionen durch einwertige Ionen, wie beispielsweise Natrium, zu ersetzen. Alternativ oder zusätzlich kann insbesondere eine, z.B. dem Ablauf des Enthärters nachgeschaltete, Einrichtung zur Verminderung der spezifischen elektrischen - bzw. ionischen - Leitfähigkeit des Wassers (ebenfalls nicht gezeigt) vorgesehen sein, welche insbesondere von einer Membrananlage, wie einer Umkehrosmoseanlage oder von einer Mikro-, Nano- oder Ultrafiltrationsanlage gebildet sein kann und die spezifische elektrische Leitfähigkeit des Wassers auf einem Wert von z.B. höchstens etwa 350 µS/cm, insbesondere von höchstens etwa 150 µS/cm, vorzugsweise von höchstens etwa 100 µS/cm, hält. Eine gleichfalls nicht dargestellte Leitfähigkeitsmeßeinrichtung, wie eine Leitfähigkeitsmeßzelle, -elektrode oder dergleichen, kann zur Überwachung der Einhaltung des jeweils gewünschten Wertes der spezifischen elektrischen Leitfähigkeit des Rohwassers dienen.

Wie weiterhin der Fig. 1 zu entnehmen ist, mündet die aus dem Speichertank 13 für die elektrochemisch aktivierte, anodische, verdünnte Wasser-/Elektrolytlösung Leitung 14 in einen weiteren Mischer 25, welcher entsprechend dem Mischer 5 (siehe auch Fig. 3) oder beliebig andersartig ausgestaltet sein kann. Der Mischer 25 dient zum möglichst homogenen Eindispergieren von amorphem Siliciumdioxid in die in der Leitung 14 geförderte elektrochemisch aktivierte Lösung und steht hierzu über eine Leitung 26, welche mit einer steuerbaren Dosierpumpe 27 ausgestattet ist, mit einem Vorratsbehälter 28 für das amorphe Siliciumdioxid in Verbindung. Bei dem verwendeten Siliciumdioxid kann es sich beispielsweise um feinpartikuläre pyrogene Kieselsäure des Typs "HDK^{®} T30" (Wacker Chemie AG, München, Deutschland), d.h. um eine synthetische, hydrophile, amorphe, flammenhydrolytisch hergestellte Kieselsäure mit einem SiO₂-Gehalt von größer 99,8%, einer Dichte des SiO₂ von 2200 g/l und einer Silanolgruppendichte von 2 SiOH/nm² handeln. Es kann der elektrochemisch aktivierten Lösung in fester oder - z.B. in Wasser - vordispergierter Form zugesetzt werden. Je nach gewünschter Viskosität des Endproduktes kann es der elektrochemisch aktivierten Lösung beispielsweise in einem Massenverhältnis von etwa 1:10 zugesetzt werden, was mittels der Dosierpumpe 27 geschieht.

Der Mischer 25 steht darüber hinaus über eine ebenfalls mit einer steuerbaren Dosierpumpe 29 ausgestattete Leitung 30 mit einem weiteren Vorratsbehälter 31 in Verbindung, welcher zur Bevorratung eines Puffers, beispielsweise Natriumcarbonat/Natriumhydrogencarbonat oder einer wäßrigen Lösung desselben, dient. Auf diese Weise kann der pH-Wert der in der Leitung 14 geförderten, elektrochemisch aktivierten, anodischen, verdünnten Wasser-/Elektrolytlösung auf einen gegenüber etwa 3 höheren pH-Wert (z.B. etwa 4 bis 7) angehoben werden, sofern dies - z.B. aus Gründen einer guten Hautverträglichkeit des fertigen Desinfektionsmittels - erwünscht ist. Die Dosierpumpe 29 steht zu diesem Zweck beispielsweise mit einer in der Leitung 32, welche das fertig homogenisierte Produkt aus dem Mischer 25 in einen Vorratsbehälter 33 überführt, aus welchem es entnommen, portioniert und verpackt werden kann, angeordneten pH-Meter (nicht gezeigt) in Verbindung, wie es mit der Strichlinie 34 angedeutet ist. Auf diese Weise läßt sich anhand des gewünschten pH-Wertes des fertigen Desinfektionsmittels die entsprechende Menge an entsprechender Pufferlösung automatisch zudosieren.

Es versteht sich im übrigen von selbst, daß anstelle der vorliegenden kontinuierlichen Verfahrensweise auch eine diskontinuierliche Verfahrensführung möglich ist, gemäß welcher die elektrochemisch aktivierte, anodische, verdünnte Wasser-/Elektrolytlösung aus der Leitung 14 beispielsweise in einen oder mehrere Rührkessel (nicht gezeigt) überführt und dort mit der entsprechenden Menge an amorphem SiO₂ und gegebenenfalls Puffer beaufschlagt wird. Ferner kann die elektrochemisch aktivierte, anodische, verdünnte Wasser-/Elektrolytlösung in unverdünnter Form oder gegebenenfalls auch in, insbesondere mit Wasser bzw. Reinwasser, verdünnter Form eingesetzt werden. Ein zur einwandfreien Desinfektion geeigneter Gehalt an desinfektiös wirksamen elekrochemischen Aktivierungsprodukten entspricht z.B. etwa 40 mg/l des Summenparameters freien Chlors, wobei selbstverständlich bedarfsweise auch höhere oder geringere Konzentrationen eingestellt werden können, was beispielsweise durch geeignete Erhöhung/Verringerung des Elektrodenstroms, der zudosierten Menge an Kochsalzlösung, der Verweilzeit der verdünnten Wasser-/Elektrolytlösung in dem Elektrolysereaktor 6 etc. geschehen kann. Schließlich muß die kontinuierliche Phase des erfindungsgemäßen Desinfektionsmittels auf der Basis der wäßrigen, hypochlorige Säure enthaltenden Lösung nicht notwendigerweise mittels elektrochemischer Aktivierung erzeugt werden, sondern kommen hierfür auch beliebige andere bekannte Herstellungsverfahren in Betracht.

## Patentansprüche

1. Verwendung einer wäßrigen, hypochlorige Säure (HOCl) enthaltenden Lösung mit einem pH-Wert zwischen 2,5 und 8, welche ferner einen Anteil an amorphem Siliciumdioxid (SiO₂) enthält, als Desinfektionsmittel.

2. Verwendung nach Anspruch 1, **dadurch gekennzeichnet, daß** das amorphe Siliciumdioxid in Form von amorphen Kieselsäuren und/oder amorphen Kieselsäureanhydriden vorliegt.

3. Verwendung nach Anspruch 1 oder 2, **dadurch gekennzeichnet, daß** das Desinfektionsmittel einen hinreichenden Anteil an amorphem Siliciumdioxid enthält, um die Viskosität der wäßrigen, hypochlorige Säure enthaltenden Lösung zu erhöhen.

4. Verwendung nach einem der Ansprüche 1 bis 3, **dadurch gekennzeichnet, daß** das Desinfektionsmittel eine gelartige Konsistenz aufweist.

5. Verwendung nach einem der Ansprüche 1 bis 4, **dadurch gekennzeichnet, daß** das Massenverhältnis der wäßrigen, hypochlorige Säure enthaltenden Lösung zu dem amorphen Siliciumdioxid zwischen 100:1 und 1:1, insbesondere zwischen 50:1 und 2:1, beträgt.

6. Verwendung nach einem der Ansprüche 1 bis 5, **dadurch gekennzeichnet, daß** die wäßrige, hypochlorige Säure enthaltende Lösung von einer elektrochemisch aktivierten, verdünnten Wasser-/Elektrolytlösung gebildet ist.

7. Verwendung nach Anspruch 6, **dadurch gekennzeichnet, daß** die wäßrige, hypochlorige Säure enthaltende Lösung ausschließlich von einer elektrochemisch aktivierten, anodischen, verdünnten Wasser-/Elektrolytlösung gebildet ist.

8. Verwendung nach einem der Ansprüche 1 bis 7, **dadurch gekennzeichnet, daß** das Desinfektionsmittel je nach Verwendung einen pH-Wert zwischen 2,5 und 7 aufweist.

9. Verwendung nach einem der Ansprüche 1 bis 8, **dadurch gekennzeichnet, daß** das Desinfektionsmittel ein Redoxpotential zwischen 1100 mV und 1360 mV, insbesondere zwischen 1150 mV und 1360 mV, vorzugsweise zwischen 1200 mV und 1360 mV, aufweist.

10. Verwendung nach einem der Ansprüche 1 bis 9, **dadurch gekennzeichnet, daß** das Desinfektionsmittel einen Summenparameter freien Chlors zwischen 10 mg/l und 70 mg/l, insbesondere zwischen 20 mg/l und 60 mg/l, vorzugsweise zwischen 30 mg/l und 50 mg/l, aufweist.

11. Verwendung nach einem der Ansprüche 1 bis 10, **dadurch gekennzeichnet, daß** das Desinfektionsmittel zur Erhöhung seines pH-Wertes einen Puffer enthält.

12. Verwendung nach Anspruch 11, **dadurch gekennzeichnet, daß** der Puffer ein solcher auf Carbonat-/Hydrogencarbonatbasis ist.

13. Verwendung nach einem der Ansprüche 1 bis 12, **dadurch gekennzeichnet, daß** die wäßrige, hypochlorige Säure enthaltende Lösung aus Reinwasser erzeugt ist.

14. Verwendung nach einem der Ansprüche 1 bis 13 zur Desinfektion von Oberflächen.

15. Verwendung nach einem der Ansprüche 1 bis 13 zur Herstellung eines Arzneimittels zur Desinfektion menschlicher und tierischer Haut.

16. Verwendung nach einem der Ansprüche 1 bis 13 zur Desinfektion von flüssigen Medien, insbesondere Wasser.

## Claims

1. Use of an aqueous, hypochlorous acid (HOCI)-containing solution having a pH-value between 2.5 and 8 which also contains a proportion of amorphous silica (SiO₂) as a disinfectant.

2. Use of according to claim 1, **characterized in that** the amorphous silica is present in the form of amorphous silicic acids and/or amorphous silicic anhydrides.

3. Use of according to either one of the claims 1 or 2, **characterized in that** the disinfectant contains a sufficient proportion of amorphous silica to increase the viscosity of the aqueous, hypochlorous acid-containing solution.

4. Use according to any one of the claims 1 to 3, **characterized in that** the disinfectant exhibits a gel-like consistency.

5. Use according to any one of the claims 1 to 4, **characterized in that** the mass ratio of the aqueous, hypochlorous acid-containing solution to the amorphous silica is between 100: 1 and 1:1, in particular, between 50:1 and 2:1.

6. Use according to any one of the claims 1 to 5, **characterized in that** the aqueous, hypochlorous acid-containing solution is constituted by an electrochemically activated, diluted water/electrolyte solution.

7. Use according to the claim 6, **characterized in that** the aqueous, hypochlorous acid-containing solution is constituted exclusively by an electrochemically activated, anodic, diluted water/electrolyte solution.

8. Use according to any one of the claims 1 to 7, **characterized in that**, depending on use, the disinfectant exhibits a pH value between 2.5 and 7.

9. Use according to any one of the claims 1 to 8, **characterized in that** the disinfectant exhibits a redox potential between 1100 mV and 1360 mV, in particular, between 1150 mV and 1360 mV, preferably between 1200 mV and 1360 mV.

10. Use according to any one of the claims 1 to 9, **characterized in that** the disinfectant exhibits a sum parameter of free chlorine between 10 mg/l and 70 mg/l, in particular, between 20 mg/l and 60 mg/l, preferably between 30 mg/l and 50 mg/l.

11. Use according to any one of the claims 1 to 10, **characterized in that** the disinfectant contains a buffer which increases the pH value thereof.

12. Use according to claims 11, **characterized in that** the buffer is based on carbonate/hydrogen carbonate.

13. Use according to any one of the claims 1 to 12, **characterized in that** the aqueous, hypochlorous and containing solution is made from pure water.

14. Use according to any one of the claims 1 to 12 for the disinfection of surfaces.

15. Use according to any one of the claims 1 to 13 for the production of a pharmaceutical disinfectant for human and animal skin.

16. Use according to any one of the claims 1 to 13 for the disinfection of liquid media, in particular, water.

## Revendications

1. Utilisation d'un acide hypochloridrique (HOCI) aqueux contenant une solution présentant une valeur de pH comprise entre 2,5 et 8 et présentant en outre une certaine teneur en dioxyde de silice (SiO₂) amorphe servant d'agent de désinfection.

2. Utilisation selon la revendication 1, **caractérisée en ce que** le dioxyde de silice amorphe est présent sous la forme d'acides de silice amorphe et/ou d'hydrides d'acide de silice amorphes.

3. Utilisation selon la revendication 1 ou 2, **caractérisée en ce que** l'agent de désinfection comporte une teneur suffisante en dioxyde de silice amorphe pour accroître la viscosité de la solution aqueuse contenant de l'acide hypochloridrique.

4. Utilisation selon l'une quelconque des revendications 1 à 3, **caractérisée en ce que** l'agent de désinfection présente une consistance de type gel.

5. Utilisation selon l'une quelconque des revendications 1 à 4, **caractérisée en ce que** le rapport massique de la solution aqueuse contenant de l'acide hypochloridrique par rapport au dioxyde de silice amorphe est compris entre 100:1 et 1:1, notamment entre 50:1 et 2:1.

6. Utilisation selon l'une quelconque des revendications 1 à 5, **caractérisée en ce que** la solution aqueuse contenant de l'acide hypochloridrique est constituée d'une solution eau/électrolyte réduite activée de façon électrochimique.

7. Utilisation selon la revendication 6, **caractérisée en ce que** la solution aqueuse contenant de l'acide hypochloridrique est constituée exclusivement d'une solution eau/électrolyte réduite, anodique, activée de façon électrochimique.

8. Utilisation selon l'une quelconque des revendications 1 à 7, **caractérisée en ce que** l'agent de désinfection présente respectivement après utilisation une valeur de pH comprise entre 2,5 et 7.

9. Utilisation selon l'une quelconque des revendications 1 à 8, **caractérisée en ce que** l'agent de désinfection présente un potentiel d'oxydo-réduction compris entre 1100 mV et 1360 mV, notamment entre 1150 mV et 1360 mV, de préférence entre 1200 mV et 1360 mV.

10. Utilisation selon l'une quelconque des revendications 1 à 9, **caractérisée en ce que** l'agent de désinfection est un paramètre additionné exempt de chlore compris entre 10 mg/l et 70 mg/l, notamment entre 20 mg/l et 60 mg/l, de préférence entre 30 mg/l et 50 mg/l.

11. Utilisation selon l'une quelconque des revendications 1 à 10, **caractérisée en ce que** l'agent de désinfection contient une solution tampon permettant d'accroître sa valeur de pH.

12. Utilisation selon la revendication 11, **caractérisée en ce que** la solution tampon est une base de carbonate/carbonate d'hydrogène.

13. Utilisation selon l'une quelconque des revendications 1 à 12, **caractérisée en ce que** la solution aqueuse contenant de l'acide hypochloridrique est produite avec de l'eau pure.

14. Utilisation selon l'une quelconque des revendications 1 à 13, servant à la désinfection de surfaces.

15. Utilisation selon l'une quelconque des revendications 1 à 13, servant à la fabrication d'un produit pharmaceutique de désinfection de la peau humaine et animale.

16. Utilisation selon l'une quelconque des revendications 1 à 13, servant à la désinfection d'agents liquides, notamment de l'eau.
